# EUROPEAN PATENT SPECIFICATION

(11) **EP 1 370 259 B1**
(45) Date of publication and mention of the grant of the patent: **17.01.2007**
(21) Application number: 02708364.1
(22) Date of filing: 22.03.2002
(51) Int. Cl.: A61K 31/4045, A61P 25/00

(54) **USE OF MELATONIN IN THE MANUFACTURE OF A MEDICAMENT FOR TREATING ATTENTION DEFICIT HYPERACTIVE DISORDER**
VERWENDUNG VON MELATONIN ZUR BEHANDLUNG DER MIT KONZENTRATIONSSCHWÄCHE EINHERGEHENDEN HYPERAKTIVITÄT
UTILISATION DE MELATONINE POUR LA PRODUCTION D'UN MEDICAMENT DESTINE A TRAITER UN TROUBLE DEFICITAIRE DE L'ATTENTION AVEC HYPERACTIVITE

(30) Priority: 22.03.2001 EP 01201094
(43) Date of publication of application: 17.12.2003
(73) Proprietor: Pooger Properties Limited, St. Helier, Jersey, Channel Islands JE1 4HH (GB)
(72) Inventor: KRUISINGA, Roelof, Johannes, Hendrik, NL-2243 HW Wassenaar (NL)
(74) Representative: Huygens, Arthur Victor
(86) International application number: PCT/EP2002/003317
(87) International publication number: WO 2002/076452

(56) References cited:
- HOBAN T F: "SLEEPLESSNESS IN CHILDREN WITH NEURODEVELOPMENTAL DISORDERS EPIDEMIOLOGY AND MANAGEMENT" CNS DRUGS, ADIS INTERNATIONAL, AUCKLAND, NZ, vol. 14, no. 1, July 2000 (2000-07), pages 11-22, XP001013140 ISSN: 1172-7047
- JADAD A R ET AL: "THE TREATMENT OF ATTENTION-DEFICIT HYPERACTIVITY DISORDER: AN ANNOTATED BIBLIOGRAPHY AND CRITICAL APPRAISAL OF PUBLISHED SYSTEMATIC REVIEWS AND METAANALYSES" CANADIAN JOURNAL OF PSYCHIATRY. REVUE CANADIENNE DE PSYCHIATRIE, CANADIAN PSYCHIATRIC ASSOCIATION, OTTAWA, CA, vol. 44, no. 10, 1999, pages 1025-1035, XP000884705 ISSN: 0706-7437

## Description

The present invention relates to the use of melatonin in the treatment of attention deficit hyperactivity disorder ("ADHD") in mammals, including humans.

Melatonin (N-acetyl-5-methoxytryptamine) is an endogenous hormone of the pineal gland, a small organ (approx. 100 mg) located in the mid-brain above the third ventricle (A.B. Lemer *et al., J. Amer. Chem. Soc.* 1958; 80:2587). The rate-limiting enzyme for its synthesis, N-acetyltransferase (NAT) is produced only during the night. Night-time values of NAT are more than 100-fold greater than daytime levels. Melatonin is also produced by extra-pineal tissues, that lightens skin color in amphibians by reversing the darkening effect of MSH (melanotropin). Melatonin has been postulated as the mediator of photic-induced anti-gonadotropic activity in photoperiodic mammals and has also been shown to be involved in thermoregulation in some ectotherms and in affecting locomotor activity rhythms in sparrows.

Melatonin, when used experimentally, is synthesised chemically and has been studied extensively in clinical and preclinical trials to examine the effects of the circadian SCN clock (A.J. Lewy et *al., Behav. Brain Res.* 1996; 73:1-2 131-4).

The suprachiasmatic nuclei of the hypothalamus control the numerous physiologic and endocrine circadian rhythms of the body, including that of rest and activity.. The circadian clock is set via a process called entrainment, which is a response of the suprachiasmatic nuclei (SCN) to photic and non-photic input of the environment (M.E. Morris *et al., Science* 1998; 279:5356 1544-1547). In all mammalian species, the SCN drives the circadian pacemaker by electrical activity through an endogenously-produced oscillation (F.K. Stephan and I. Zucker, *Proc. Natl. Acad Sci. USA* 1972; 69(6):1583-1586). Synthesis and secretion of endogenous melatonin is controlled by enzymes secreted by the hypothalamus which are activated by darkness and depressed by environmental light (S.M. Armstrong, *in: Pineal Research Reviews.* New York: Alan R. Liss, 1989(7):157-202). Exactly how melatonin induces sleep is not clear, but it is probably not through a direct hypnotic effect. In patients with jet lag or circadian rhythm disorders, endogenous melatonin secretion does not correspond to the social or solar sleep-wake cycles imposed by their surroundings, and they experience sleep disruption (C. Liu *et al. Neuron* 1997; 19(1) 91-102). Administration of exogenous melatonin appears to re-set the body to the environmental clock and allow patients to normalize physiologic and behavioural sleep patterns. Exogenous melatonin maximally advances delayed rhythms when administered before endogenous melatonin levels begin to increase in the evening hours. In addition to circadian phase-shifting effects, melatonin has been shown to decrease nocturnal core body temperature, which helps to facilitate sleep. To date, pharmacological tolerance to melatonin has not been described.

Melatonin is involved in other physiologic processes besides the sleep-wake cycle. Secretion of melatonin from the pineal gland is highest during the pediatric years and tends to decrease with age. This age-related secretion performs important endocrine functions. It is thought that higher pre-pubertal melatonin levels are responsible for keeping the hypothalamic-pituitary-gonadal axis in quiescence, and that decreasing melatonin levels with age play a role in the onset of adolescence and sexual maturation. Melatonin receptors have been found in all male and female sexually responsive tissues, indicating that melatonin has a significant role in normal reproductive capacity. Exogenous melatonin can suppress the release of gonadotropin releasing hormone and lutenizing hormone, leading to anovulation and changes in steroid responsive tissues, especially in higher doses. In woman contraceptive activity has been noted when melatonin is given in combination with norethindrone.

Melatonin also exhibits immunostimulatory and antioxidant actions. In neurodegenerative disease models, melatonin appears to neutralize oxidizing free radicals, specifically by preventing the reduction of antioxidant enzyme activity, and reducing beta-amyloid mediated lipid peroxidation of cell membranes. These actions appear to decrease apoptosis of neuronal cells. Further research is needed to determine if melatonin may preserve function in neurologic diseases where free radicals have been implicated as partially causative of the conditions. In epilepsy, the rise and fall of endogenous melatonin levels may influence seizure activity; melatonin appears to have both anti-convulsant and pro-convulsant effects. Preliminary *in vitro* studies have shown melatonin may augment some chemotherapy regimens, decrease free-radical mediated toxic side effects of some chemotherapy agents, and have antiproliferative effects on some tumors. Melatonin may also stimulate the activity of natural killer (NK) cells, lymphocytes, and various cytokines. Further study in well-controlled trials should answer further questions regarding melatonin's neurologic, immunologic, and oncostatic activities (*Clinical Pharmacology Online*).

WO 88/07370 discloses compositions and methods of effecting contraception and control of breast cancer involving the use of melatonin, whereas WO 91/12007 discloses a method of treating human females who suffer from pre-menstrual syndrome (PMS) which comprises administering melatonin in sufficient doses to relieve the symptoms of PMS.

A.J. Lewy *et al.* disclose the treatment of circadian rhytm disorders involving the use of melatonin in various aspects. See US 5,242,941; US 5,420,152; US 5,591,768; US 5,716,978; and US 6,069,164. Likewise, US 5,707,652 discloses a dosage form comprising a sustained release melatonin formulation, as well as a method of treating circadian rhythm disorders which involves oral administration of such formulation to produce a normal melatonin pattern when the normal pattern has been disrupted or is missing.

J.E. Jan *et al.,* Developmental Medicine and Child Neurology, 36:97-107 (1994), describe the treatment of severe, chronic sleep disorders with melatonin in fifteen children, most of whom were neurologically multiply disabled. The children were treated with 2 to 10 mg of oral melatonin, given at bedtime. The health, behavioural and social benefits were significant, and there were no adverse side-effects. While the response was not always complete, it was reported that the study clearly showed that melatonin has an important role in the treatment of certain types of chronic sleep disorders.

J.E. Jan *et al.,* J. Pineal Res. 29:34-39 (2000), report the first study to examine effective dose of controlled-release (CR) melatonin in children with chronic sleep-wake cycle disorders. The average final CR melatonin dose in the 42 children was 5.7 mg (2-12 mg). The studies showed that the fast-release melatonin was most effective when there was only delayed sleep onset, but CR formulations were more useful for sleep maintenance. Children appeared to require higher doses than adults.

As described in EP-A-0 896 536, ADHD is a condition affecting a significant proportion of children and which is manifest by learning difficulties, restlessness, inability to settle to any task, argumentativeness, low frustration tolerance and aggressive conduct. In the past, a traditional method of treating such children was by administration of psycho-stimulant such as methyl phenidate. While psychostimulants are useful in increasing attention spans, they have major side-effects, including loss of appetite and insomnia and do not deal with the problems of hyperactivity.

Said EP-A-O 896 536 discloses the use of lofexidine, 2-[α-(2,6-dichlorophenoxy)ethyl-Δ²-imidazole, in the manufacture of a medicament for treating ADHD, which reportedly does not incur the same level of side-effects as clonidine. The latter compound (see Hunt *et al.,* Journal of the American Academy of Child Adolescent Psychiatry 24 (1995)) hals been shown to be effective in treating ADHD, but it may also cause hypotension and a high level of sedation as a side-effect. It is stated in said EP reference that while a measure of sedation can be useful in the treatment of hyperactive children, it does not assist in increasing attention span.

Furthermore, WO 00/16777 discloses the use of certain pyrido[1,2-a]-pyrazine compounds, also described as bis-azabicyclic compounds, in the treatment of Parkinson's disease, ADHD, and microadenomas in mammals.

The present invention is based on the discovery that melatonin has usefulness in the treatment of ADHD.

Accordingly, there is provided the use of at least one of melatonin, a melatonin analogue, or a pharmaceutically acceptable salt of melatonin or said melatonin analogue, in the preparation of a medicament for the treatment of ADHD in mammals, in particular human beings.

As used herein, a "melatonin analogue" is meant to indicate a compound or substance exhibiting high affinity for melatonin receptors.

The medicament for the treatment of ADHD comprising melatonin and/or a melatonin analogue and/or a pharmaceutically acceptable salt thereof as an active ingredient is suitably to be administered to the mammal in the form of a pharmaceutical composition. The administration may be by way of oral or parenteral administration.

The medicament can be administered in conventional form for oral administration, e.g. as tablets, lozenges, dragees and capsules. However, for the administration of the drug to children, which is likely to be its major use, it may be preferred to formulate the composition as an oral liquid preparation such as a syrup, a nasal spray, or a suppository. The medicament can also be administered parenterally, e.g. by intramuscular or subcutaneous injection, using formulations in which the medicament is employed in a saline or other pharmaceutically acceptable, injectable composition.

An amount effective to treat the disorder hereinbefore described depends on the usual factors such as the nature and severity of the disorder being treated, the weight of the mammal, the specific compound(s) of choice, and considerations and preferences of the prescriber. The amount of active ingredient(s) to be administered usually will be in the range of nanograms to 50 mg or more per dose. However, a unit dose will normally contain 1 to 1000 mg, suitably 1 to 500 mg, for example an amount in the range of from 2 to 400 mg such as 2, 5, 10, 20, 30, 40, 50, 100, 200, 300 and 400 mg of the active ingredient. Unit doses will normally be administered once or more than once per day, for example 1, 2, 3, 4, 5 or 6 times a day, more usually 1 to 4 times a day, such that the total daily dose is normally in the range, for a 70 kg adult, of 1 to 1000 mg, for example 1 to 500 mg, that is in the range of approximately 0.01 to 15 mg/kg/day, more usually 0.1 to 6 mg/kg/day, for example 1 to 6 mg/kg/day.

It is greatly preferred that melatonin and/or a melatonin analogue and/or a pharmaceutically acceptable salt thereof according to the invention is administered in the form of a unit-dose composition, such as a unit dose oral, such as sub-lingual, rectal, topical or parenteral (especially intravenous) composition.

Such compositions are prepared by admixture and are suitably adapted for oral or parenteral administration, and as such may be in the form of tablets, capsules, oral liquid preparations, powders, granules, lozenges, reconstitutable powders, injectable and infusable solutions or suspensions or suppositories. Orally administrable compositions are preferred, in particular shaped oral compositions, since they are more convenient for general use. The preparation of such compositions is well known to people skilled in the art and can be optimized in a routine way without exerting inventive skill and without undue experimentation.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art.

Suitable fillers for use include, mannitol and other similar agents. Suitable disintegrants include starch derivatives such as sodium starch glycollate. Suitable lubricants include, for example, magnesium stearate.

These solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

Oral formulations further include controlled release formulations which may also be useful in the practice of this invention. The controlled release formulation may be designed to give an initial high dose of the active material and then a steady dose over an extended period of time, or a slow build up to the desired dose rate, or variations of these procedures. Controlled release formulations also include conventional sustained release formulations, for example tablets or granules having an enteric coating.

Nasal spray compositions are also a useful way of administering the pharmaceutical preparations of this invention to patients such as children for whom compliance is difficult. Such formulations are generally aqueous and are packaged in a nasal spray applicator which delivers a fine spray of the composition to the nasal passages.

Suppositories are also a traditionally good way of administering drugs to children and can be used for the purposes of this invention. Typical bases for formulating suppositories include water-soluble diluents such as polyalkylene glycols and fats, e.g. cocoa oil and polyglycol ester or mixtures of such materials.

For parenteral administration, fluid unit dose forms are prepared containing the compound and a sterile vehicle. The compound, depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle.

Parenteral suspensions are prepared in substantially the same manner except that the compound is suspended in the vehicle instead of being dissolved and sterilised usually by exposure to ethylene oxide before suspending in the sterile vehicle. Advantageously, a surfactant or wetting agent is included in the composition to facilitate uniform distri- bution of the compound of the invention.

As is common practice, the compositions will usually be accompanied by written or printed directions for use in the medical treatment concerned.

In the treatment of ADHD patients in accordance with the invention, melatonin or a melatonin analogue can be used alone or together with other active materials. The latter materials are preferably chosen such that either their activiy is enhanced, preferably in a synergistic way, or undesired side-effects are suppressed by melatonin and/or its analogue. For example, melatonin or its analogue which can be used in conjunction with the medicament additionally contains one or more substances selected from the group of stimulants, hormones, analogues of such hormones, phyto-hormones, analogues of such phyto-hormones like phyto estrogen, and anti-oxidants like phyto vitamins c and e, flavonoids.

Preliminary investigations show the following dose rates. For the occasional self-treatment of mild insomnia in adults: 0.3 to 3 mg oral or sublingual dosage (PO), in the evening hours approximately 1 to 2 hours before habitual bedtime. May take up to 6 mg PO if needed. For the adjunctive treatment of insomnia related to major depression: Adults: 5 to 10 mg oral extended release formulations (PO) taken 1 to 2 hours prior to habitual bedtime. In one 4-week placebo-controlled study of 19 patients with major depressive disorder treated with fluoxetine, the sub-group of 10 patients who received concomitant slow-release melatonin at 9 pm for sleep reported significantly improved sleep quality scores versus the patients receiving fluoxetine alone. Melatonin treatment avoided the need for additional sleep medications. No differences in the rates of improvement of depressive symptoms or side effects were reported between the two groups. (Dolberg et al; 1998)

For the treatment of delayed sleep phase syndrome resulting from circadian rhythm disruption, including patients with autism, blindness, Rett's syndrome, or developmental disabilities in adults: Doses of 5 to 7 mg oral immediate release formulations (PO) once daily at bedtime have been used in the blind to entrain circadian rhythms to a 24-hour day. (Sack et al; 1991); in children: Doses of 2.5 to 7.5 mg PO once daily before expected bedtime have been used. The average onset of sleep occurred within 1 hour of melatonin administration. Most children were on concomitant anti-convulsant therapies. Melatonin was administered nightly for up to 4 weeks and appeared to be well tolerated. The long-term effects of chronic melatonin use in pediatric patients are unknown. (Chase & Gidal; 1997, McArthur & Budden; 1998) Although Palm et al (1999) and Jan et al (2000) published reports on children who received melatonin for several years without adverse effects. However, doses administered would, to a large extent, depend upon the method of administration.

In a pilot study, nine patients in the age ranging from 6 to 14 years were run through a protocol to determine feasibility. A sample of four sleep logs, illustrating response at each of the protocol was determined. During working with the children it became apparent that the effect of melatonin on sleep latency could only be measured in children who also received sleep hygiene. Three sleep logs illustrate erratic sleep patterns before melatonin, stable sleep with long latency at baseline, and then sleep on melatonin at follow up. Patients tolerated the protocol, and parents were able to reliably complete the sleep logs. Some parents had to be trained to do so over several visits. Parents whose children fell asleep extremely late, could not tolerate staying up to find out when they actually fell asleep, and had to be excluded. Statistical analysis of the small sample revealed no differential carryover effect from placebo to melatonin or vice versa. There was a significant difference in response (p=.03) between the melatonin and placebo. The CGI data were not significant.

Although the invention has been described primarily as a therapy for children, it can also be used for adults, although dosage rates may be different in the case of adults. Adaptation and optimization of dosages can be readily achieved by skilled persons without undue experimentation.

The following Examples which are not intended to limit the invention in any respect, show some useful pharmaceutical formulations of melatonin in the treatment of ADHD.

### Example 1

A tablet is formulated containing:

| | |
|---|---|
| Melatonin | 5.0 mg |
| Mannitol | 20.0 mg |
| Calcium hydrogen phosphate | 42.0 mg |
| Sodium starch glycollate | 5.0 mg |
| Talc | 2.5 mg |
| Magnesium stearate | 0.5 mg |

The dissolution profile of this tablet results in a melatonin release of more than 90% within 30 minutes. The disintegration time is very short and the materials meet the requirements for a dispersible tablet (Ph.Eur).

### Example 2

A capsule is formulated containing:

| | |
|---|---|
| Melatonin | 5.0 mg |
| Mannitol | 20.0 mg |
| Calcium hydrogen phosphate | 66.0 mg |
| Ethylcellulose | 1.0 mg |
| Sodium starch glycollate | 5.0 mg |
| Talc | 2.5 mg |
| Magnesium stearate | 0.5 mg |
| HPMC Capsule | 37.5 mg |

The dissolution profile of this capsule results in a direct release of 1.8 mg of melatonin (90% of 2 mg) within 30 minutes, and a sustained release of the remaining 3 mg within 6 hours.

## Claims

1. Use of at least one of melatonin, a melatonin analogue, or a pharmaceutically acceptable salt of melatonin or said melatonin analogue, in the preparation of a medicament for the treatment of attention deficit hyperactivity disorder (ADHD) in a mammal, especially a human being.

2. Use as daimed in claim 1 wherein the melatonin or melatonin analogue is employed in an amount of from 0.005 to 1.00 mg/kg in treating ADHD.

3. Use as claimed in any one of the preceding claims wherein the medicament is formulated as a controlled release preparation.

4. Use as daimed in any one of the preceding claims wherein the medicament is formulated as a solid oral formulation.

5. Use as claimed in any one of the preceding claims wherein the medicament additionally contains one or more substances selected from the group of stimulants, hormones, analogues of such hormones, phyto-hormones, analogues of such phyto-hormones, and anti-oxidants.

## Patentansprüche

1. Verwendung mindestens eines Melatonins, eines Melatoninanalogons oder eines pharmazeutisch verträglichen Salzes von Melatonin oder des Melatoninanalogons zur Herstellung eines Medikaments zur Behandlung des Aufmerksamkeitsdefizit-Hyperaktivitätssyndroms (ADHD) in einem Säuger, insbesondere einem Menschen.

2. Verwendung nach Anspruch 1, wobei das Melatonin oder Melatoninanalogon in einer Menge von 0,005 bis 1,00 mg/kg bei der Behandlung von ADHD verwendet wird.

3. Verwendung nach einem der vorstehenden Ansprüche, wobei das Medikament als ein Mittel zur kontrollierten Freisetzung formuliert ist.

4. Verwendung nach einem der vorstehenden Ansprüche, wobei das Medikament als eine feste Oralformulierung formuliert ist.

5. Verwendung nach einem der vorstehenden Ansprüche, wobei das Medikament zusätzlich eine oder mehrere Substanzen enthält, die aus Stimulantien, Hormonen, Analoga solcher Hormone, Phytohormonen, Analoga solcher Phytohormone und Antioxidationsmitteln ausgewählt sind.

## Revendications

1. Utilisation d'au moins un composé parmi la mélatonine, un analogue de mélatonine, ou un sel pharmaceutiquement acceptable de mélatonine ou dudit analogue de mélatonine, dans la préparation d'un médicament destiné au traitement du trouble déficitaire de l'attention avec hyperactivité (ADHD) chez un mammifère, en particulier chez un être humain.

2. Utilisation selon la revendication 1 dans laquelle la mélatonine ou l'analogue de mélatonine est employé en une quantité de 0,005 à 1,00 mg/pour traiter l'ADHD.

3. Utilisation selon l'une quelconque des revendications précédentes dans laquelle le médicament est formulé en préparation à libération contrôlée.

4. Utilisation selon l'une quelconque des revendications précédentes dans laquelle le médicament est formulé en formulation orale solide.

5. Utilisation selon l'une quelconque des revendications précédentes dans laquelle le médicament contient en plus une ou plusieurs substances choisies dans le groupe des stimulants, des hormones, des analogues de ces hormones, des phytohormones, des analogues de ces phytohormones et des antioxydants.
